# EUROPEAN PATENT SPECIFICATION

(11) **EP 3 003 339 B1**
(45) Date of publication and mention of the grant of the patent: **22.03.2023**
(21) Application number: 14728500.1
(22) Date of filing: 28.05.2014
(51) Int. Cl.: A61K 31/05, A61K 31/12, A61K 31/7048, A61K 36/9066, A61K 45/06, A61P 19/02, A61K 36/76, A61K 36/815, A61K 36/82, A61K 36/88, A61K 36/9068, A61K 31/352, A61K 31/353, A61K 36/185, A61K 36/38, A61K 36/324, A61K 36/53, A61K 36/71, A23L 33/00, A23L 33/10, A61K 36/28, A61P 19/00, A61P 19/08, A61P 29/00, A61P 39/06, A61P 43/00

(54) **OLEUROPEIN FOR USE IN CARTILAGE BREAKDOWN**
OLEUROPEIN ZUR VERWENDUNG BEI KNORPELABBAU
OLEUROPEIN UTILISÉE EN RELATION AVEC LA DÉGRADATION CARTILAGINEUSE

(30) Priority: 29.05.2013 EP 13169656
(43) Date of publication of application: 13.04.2016
(60) Divisional application: 23155898.2
(73) Proprietor: Société des Produits Nestlé S.A., 1800 Vevey (CH)
(72) Inventor: HORCAJADA, Marie Noëlle, 01170 Echenevex (FR); MEMBREZ, Fanny, 1053 Cugy (CH); OFFORD CAVIN, Elizabeth, 1820 Montreux (CH)
(74) Representative: Elkington and Fife LLP
(86) International application number: PCT/EP2014/061017
(87) International publication number: WO 2014/191447

(56) References cited:
- EP-A1- 1 982 706
- WO-A1-2009/132807
- WO-A1-2014/161872
- US-A1- 2004 121 024
- US-A1- 2006 193 931
- US-A1- 2010 056 463
- US-A1- 2013 115 202

## Description

### Technical field of the invention

The present invention relates to joint health and in particular to use of a composition comprising oleuropein for use to inhibit or decrease cartilage breakdown, wherein the cartilage breakdown takes place in the context of a pathology with no inflammatory component.

### Background of the invention

Osteoarthritis (OA) is a high prevalence disease with an important socioeconomical impact. It is a degenerative disease of the articular cartilage of the joint, and is the most common form of arthritis, affecting 10% of the adult population. OA is the leading cause of disability in elderly and health care expenses throughout the world.

The progressive breakdown and loss of the articular cartilage belongs to the main features of the pathology, accompanied by changes to other joint structures such as synovial membrane proliferation, sclerosis and thickness of subchondral bone, osteophyte formation at joint margin, ligament laxity and muscle atrophy, all of which contribute to the clinical symptoms of OA. These symptoms include severe pain, stiffness, loss of joint motion and disability.

Although there is an increase of individuals suffering from OA, there is still no cure and current medical therapies remain only palliative, focused on alleviation of symptoms. For example, pain and inflammation are treated using analgesics (such as acetaminophen) and non-steroidal anti-inflammatory drugs (NSAIDs). Furthermore, the use of these drugs is often associated with side effects such as gastrointestinal or cardiovascular risks.

US 2010/056463 A1 discloses novel compositions comprising hydroxytyrosol and/or oleuropein (I) and at least one additional component e.g. selected from the group of ligustilide, oleuropein aglycone, magnolol, honokiol, genistein, resveratrol, EGCG, magnolia bark extract, cashew fruit extract and Glycyrrhiza foetida as well as to the use of these compositions as a medicament, in particular as a medicament for the treatment, co-treatment or prevention of inflammatory disorders.

US 2013/115202 A1 discloses methods of treating a neuro-inflammation disorder in a subject, comprising administering to a subject in need thereof an effective amount of a composition comprising a flavonoid, or a structurally related analogue, olive kernel extract, hydroxytyrosol, and berberine, and, optionally, one or more ingredients selected from the group consisting of a sulfated proteoglycan, oleocanthal, a CRH antagonist, S adenosylmethionine, a histamine 1 receptor antagonist, a histamine 3 receptor agonist, emu oil, oregano oil, grape seed oil, aloe extract, biotin, and selenium. Certain of the present compositions are useful in protecting against or treating neuro-inflammation associated with allergies, Alzheimer's disease (AD), atherosclerosis, asthma, Autistic Spectrum Disorders (ASD).

WO 2009/132807 A1 particular compositions comprising certain combinations of the main olive polyphenols, such as for instance oleuropein and/or ligstroside in combination with hydroxytyrosol and/or tyrosol characterized in that the ratio of non-hydrolysed polyphenols (oleuropein and/or ligstroside) to hydrolysed polyphenols (hydroxytyrosol and/or tyrosol) is within certain thresholds which unexpectedly provide certain health benefits as compared to other compositions of olive polyphenols already known in the art. In a particular embodiment of the present invention the compositions contain standardized quantities for at least two of said olive polyphenols.

EP1982706 A1 the use of hydroxytyrosol for inducing or enhancing cartilage repair or cartilage regeneration.

WO 2014/161872 A1 use of a composition for maintenance of bone and/or cartilage health or prevention, alleviation and/or treatment of bone and/or cartilage disorders.

US 2006/193931 A1 provides a nutritional composition and a pharmaceutical composition to be used for humans or animals comprising as active compound the oleuropein compound or one derivative thereof.

US 2004/121024 A1 involves a composition for oral ingestion that contains effective amounts of Glucosamine sulfate, Nettle Leaf, Quercetin, curcumin extract/curcuminoids, Selenium, Zinc, Vitamin C (calcium ascorbate) and Grape Seed Extract, as well as other ingredients and healthy filler ingredients.

Hence, improved compositions for use in therapy for OA would be advantageous, and in particular more effective and safe compostitions are desired.

### Summary of the invention

Anti-inflammatory and antioxidant properties of oleuropein and hydroxytyrosol have been observed previously. However, the inventors have surprisingly demonstrated that these compounds also have anti-catabolic effects on cartilage.

An object of the present invention therefore relates to providing compositions for use in improving joint health. In particular, it is an object of the present invention to provide compositions which improve joint health by inhibiting or decreasing cartilage breakdown, and solves the above mentioned problems of the prior art with regards to side effects such as gastrointestinal and/or cardiovascular risks.

Thus, one aspect of the invention relates to a composition comprising oleuropein for use to inhibit or decrease cartilage breakdown, wherein the cartilage breakdown takes place in the context of a pathology with no inflammatory component.

### Brief description of the figures

Figure 1A shows ADAMTS-5 mRNA expressions relative to GAPDH in absence of ILia which mimics a normal state of the cells. Conditions were statistically compared to CTL-.
Figure 1B shows ADAMTS-5 mRNA expressions relative to GAPDH in presence of ILia which mimics a disease state of the cells. Conditions were statistically compared to IL1a except IL1a which was compared to CTL- (validation of the experiment).
Figure 2A shows COX-2 mRNA expressions relative to GAPDH in absence of ILia which mimics a normal state of the cells. Conditions were statistically compared to CTL-.
Figure 2B shows COX-2 mRNA expressions relative to GAPDH in presence of ILia which mimics a disease state of the cells. Conditions were statistically compared to IL1a except IL1a which was compared to CTL- (validation of the experiment).
Figure 3A shows MMP-13 mRNA expressions relative to GAPDH in absence of ILia which mimics a normal state of the cells. Conditions were statistically compared to CTL-.
Figure 3B shows MMP-13 mRNA expressions relative to GAPDH in presence of ILia which mimics a disease state of the cells. Conditions were statistically compared to IL1a except IL1a which was compared to CTL- (validation of the experiment).
Figure 4 shows histological sections of the knee of guinea pigs (hematoxylin, fast green and safranin-O staining). 4A and 4B show Group A (oleuropein); 4C and 4D show Group C (rutin); 4E and 4F show Group D (rutin and curcumin); 4G and 4H show group B (Control).
Figure 5 shows total OA score in each group.
Figure 6A shows concentration of inflammatory PGE2 biomarker in plasma. 6B shows the correlation between PGE2 levels in plasma at weeks 4 and 35 and corresponding OA score.
Figure 7 shows concentration of Coll 2-1 NO2 biomarker in plasma linked to collagen 2 breakdown over time.
Figure 8A shows concentration of Coll 2-1 NO2 biomarker in plasma linked to collagen 2 breakdown.
Figure 8B shows the correlation between concentration of Coll 2-1 NO2 biomarker in plasma at weeks 4 and 35 and corresponding OA score.
Figure 9 shows concentration of Fibulin 3-1 biomarker in plasma linked to hypertrophic action of chondrocytes in OA patients.
Figure 10A shows concentration of Fibulin 3-1 biomarker in plasma linked to hypertrophic action of chondrocytes in OA patients.
Figure 10B shows the correlation between concentration of Fibulin 3-1 biomarker in plasma at weeks 4 and 35 and corresponding OA score.

### Detailed description of the invention

### Definitions

Prior to discussing the present invention in further details, the following terms and conventions will first be defined:
In the context of the present invention, mentioned percentages are weight/weight percentages unless otherwise stated.

The term "and/or" used in the context of the "X and/or Y" should be interpreted as "X", or "Y", or "X and Y".

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 4 to 9, from 3.6 to 4.6, from 3.5 to 9.9, and so forth.

All references to singular characteristics or limitations of the present invention shall include the corresponding plural characteristic or limitation, and vice versa, unless otherwise specified or clearly implied to the contrary by the context in which the reference is made.

Unless defined otherwise, all technical and scientific terms used herein have the same meaning as commonly understood by one of ordinary skill in the art.

### Composition for use

Oleuropein is a polyphenol which has previously been shown to have beneficial effects on inflammation and bone metabolism. Hydroxytyrosol is a related compound, being a metabolite of oleuropein, and has also been shown to have these effects. The inventors have now surprisingly shown that oleuropein and hydroxytyrosol each also have beneficial effects on cartilage breakdown.

Joint disease may be accompanied by inflammation to a greater or lesser degree. In some diseases, the inflammation is an overriding component, such as for example in Rheumatoid artritis (RA). In other diseases, such as for example OA, the inflammation does not appear as prominently. However, both diseases have a catabolic component in which the articular cartilage is broken down.

The present inventors have shown that the provision of oleuropein can prevent cartilage breakdown in an animal model of OA. Hydroxytyrosol has also been shown to inhibit catabolic enzymes in a cartilage model system.

Thus, the invention in a first aspect relates to a composition comprising oleuropein for use to inhibit or decrease cartilage breakdown, wherein the cartilage breakdown takes place in the context of a pathology with no inflammatory component.

The use to prevent or treat cartilage breakdown is synonymous with use to inhibit or decrease cartilage breakdown.

Embodiments of the invention thus include a composition comprising oleuropein for use to inhibit or decrease cartilage breakdown, wherein the cartilage breakdown takes place in the context of a pathology with no inflammatory component.

Yet another embodiment of the invention includes a composition comprising oleuropein and hydroxytyrosol for use to inhibit or decrease cartilage breakdown, wherein the cartilage breakdown takes place in the context of a pathology with no inflammatory component.

Further embodiments of the invention include a composition for use according to the invention, wherein the composition further comprises curcumin, as well as a composition for use according to the invention, wherein the composition further comprises quercetin.

In embodiments of the invention the composition for use according to the invention comprises oleuropein, as well as one or more further polyphenol, for example one or more further polyphenols selected from the group consisting of curcumin, and quercetin.

One embodiment of the invention relates to a composition for use according to the invention, wherein the composition comprises oleuropein and further comprises curcumin.

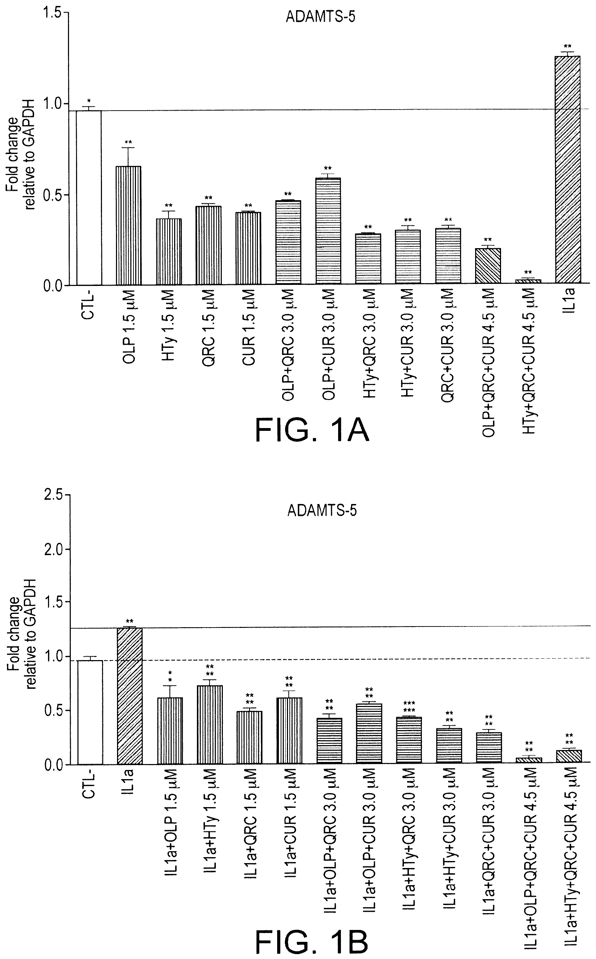

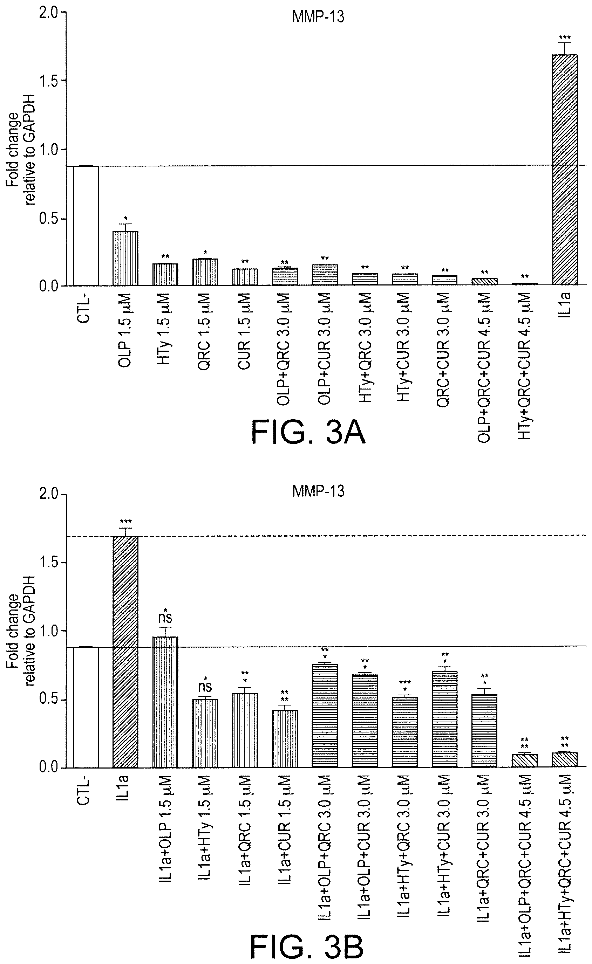

The combination of oleuropein and curcumin has both been shown to inhibit catabolic enzymes in an *in vitro* model of cartilage (see figures 1 and 3, Example 1).

Another embodiment of the invention relates to a composition for use according to the invention, wherein the composition comprises oleuropein and further comprises quercetin.

The combination of oleuropein and quercetin has both been shown to inhibit catabolic enzymes in an *in vitro* model of cartilage (see figures 1 and 3, Example 1).

In yet another embodiment, the composition for use according to the invention comprises oleuropein, and further comprises curcumin and quercetin.

The combination of oleuropein + curcumin + quercetin has been shown to have synergistic and specific effects on the catabolic enzymes active in cartilage breakdown (see figures 1 and 3, Example 1).

### Ingredients- main bioactive compounds

The oleuropein, curcumin and quercetin are the main bioactive molecules according to present invention. They may be from any suitable source. In preferred embodiments, oleuropein, curcumin and quercetin are obtained from plant sources. Oleuropein may for example be obtained from the olive plant.

### Ingredients- further bioactive compound

The compositions for use according to the invention may also comprise at least one further bioactive compound, such as a compound selected from the group consisting of antioxidants, anti-inflammatory compounds, glycosaminoglycans, prebiotics, fibres, probiotics, fatty acids, minerals, trace elements and/or vitamins.

The term "bioactive" in the context of the present application means that the compound contributes to the health of an individual, or has an effect on the human body, beyond that of meeting basic nutritional need.

The at least one further bioactive compound may be from a natural source. Thus the compounds may be from extracts of plants, animals, fish, fungi, algae, microbial fermentation. Minerals are considered as from natural source also within this definition.

### Anti-inflammatory compounds and/or antioxidants

### Combination with anti-inflammatory compounds

The present invention relates in one aspect to compositions for use to inhibit or decrease cartilage breakdown, wherein the cartilage breakdown takes place in the context of a pathology with no inflammatory component.

In one embodiment of the invention, the compositions according to the invention are for combined use with at least one anti-inflammatory compound.

The at least one anti-inflammatory compound may be administered in combination with a composition of the invention, for example sequentially or simultaneously.

The at least one anti-inflammatory compound may be provided in the same composition as the composition of the invention, or may be provided in separate compositions, for example in a kit of parts, or a series of interacting products.

If the composition for use according to the invention and the at least one anti-inflammatory compound are presented in one or more separate compositions, they may be mixed prior to administration, or may be administered simultaneously or sequentially.

In further embodiments, the composition for use according to the invention comprises at least one anti-inflammatory compound as an at least one further bioactive compound. In other preferred embodiments, the composition for use according to the invention comprises at least one antioxidant as an at least one a further bioactive compound.

The at least one anti-inflammatory and/or antioxidant compound may be any suitable compound with anti-inflammatory and/or antioxidant effect.

It is noted that several anti-inflammatory compounds also have antioxidant effect. A compound may be selected for inclusion based on it having either or both properties.

Examples of anti-inflammatory compounds which may be included in a composition of the invention are anti-inflammatory compounds from natural sources.

Thus, one embodiment of the invention relates to a composition for use according to the invention, wherein the at least one anti-inflammatory compound comprises omega-3 polyunsaturated fatty acids, which may for example be extracted from fish.

Other embodiments of the invention relate to compositions for use according to the invention, wherein the at least one anti-inflammatory compound comprised is at least one plant extract.

The plant extract may comprise for example flavonoids, polyphenols, proanthocyanins, lipids (such as avocado soybean unsaponifiables, omega-3 polyunsaturated fatty acids).

Plant extracts may be from one or more plants selected from the group consisting of devil's claw (*Harpagophytum procumbens*), Rosmarinus officinalis, hyssop, ginger *(Zingiber officinale),* turmeric (*Curcuma longa*), Arnica Montana, willow bark, pomegranate *(Punica granatum),* green tea *(Camellia sinensis),* cat's claw *(Uncaria tometosa* and *Uncaria guianensis),* Indian olibaum (*Boswelia serrata*), and pineapple bromelain (*Ananas comosus*)*,* Black seed (*Nigella sativa*), St. John's wort, hyperforin, Goldenseal, German Chamomile (*Matricaria recutita*).

The at least one antioxidant which may be included in a composition of the invention may be selected from the group consisting of astaxanthin, carotenoids, coenzyme Q10 ("CoQ10"), flavonoids, glutathione, Goji (wolfberry), hesperidin, lactowolfberry, lignan, lutein, lycopene, polyphenols, selenium, vitamin A, vitamin C, vitamin E, zeaxanthin.

In preferred embodiments, the antioxidant is from a natural source, such as for example a plant extract.

### Glycosaminoglycans

Glycosaminoglycans are large, heavily charged molecules which are present in healthy cartilage. Examples of glycosaminoglycans are glucosamine and chondroitin sulphate.

Thus, one embodiment of the composition for use according to the invention comprises glucosamine and/or chondroitin sulphate.

### Prebiotics

In some embodiments, the compositions for use according to the invention include one or more prebiotics. Non-limiting examples of prebiotics include acacia gum, alpha glucan, arabinogalactans, beta glucan, dextrans, fructooligosaccharides, fucosyllactose, galactooligosaccharides, galactomannans, gentiooligosaccharides, glucooligosaccharides, guar gum, inulin, isomaltooligosaccharides, lactoneotetraose, lactosucrose, lactulose, levan, maltodextrins, milk oligosaccharides, partially hydrolyzed guar gum, pecticoligosaccharides, resistant starches, retrograded starch, sialooligosaccharides, sialyllactose, soyoligosaccharides, sugar alcohols, xylooligosaccharides, their hydrolysates, or combinations thereof.

### Fibre

The compositions for use according to the invention may include fibre. The fibre may be soluble fibre, insoluble fibre or a combination thereof. Soluble fibres may include, for example, fructooligosaccharides, acacia gum, inulin, agar-agar, an alginate, carob bean, carragheenan, gum arabic, guar gum, karaya gum, pectin or xanthan gum, etc, these soluble fibres being in a hydrolysed or non-hydrolysed form. Insoluble fibres may include, for example, pea outer fibre.

### Probiotics

The compositions for use according to the invention may include one or more probiotics. Non-limiting examples of probiotics include *Aerococcus, Aspergillus, Bacteroides, Bifidobacterium, Candida, Clostridium, Debaromyces, Enterococcus, Fusobacterium, Lactobacillus, Lactococcus, Leuconostoc, Melissococcus, Micrococcus, Mucor, Oenococcus, Pediococcus, Penicillium, Peptostrepococcus, Pichia, Propionibacterium, Pseudocatenulatum, Rhizopus, Saccharomyces, Staphylococcus, Streptococcus, Torulopsis, Weissella,* nonreplicating microorganisms, and combinations thereof.

### Minerals and trace elements

Examples of minerals and trace elements which may be comprised in a composition according to the invention include mineral elements and trace elements such as calcium, magnesium, sodium, potassium, phosphorus, copper, zinc, iron, selenium, chromium and molybdenum, and combinations thereof.

### Vitamins

Compositions for use according to the invention may also comprise one or more further vitamins selected from vitamin A, vitamin D, vitamin E, vitamin K, vitamin C, folic acid, thiamine, riboflavin, vitamin B6, vitamin B12, niacin, biotin and pantothenic acid.

### Amounts

A composition for use according to the invention may comprise an amount of each of the main bioactive ingredients oleuropein, curcumin and quercetin from 0.01 mg to about 1 g, preferably from 0.1 mg to 1 g, even more preferably from 1 mg to about 1 g of each component per serving.

### Nutritional compositions

The compositions for use according to the invention may be nutritional compositions or pharmaceutical compositions, and may be for human or veterinary use.

Thus, in preferred embodiments, the composition for use according to the invention is a nutritional composition.

By "nutritional composition" is meant in the context of the present invention any composition which is a source of nutrition to an individual.

The nutritional products or compositions of the invention may be a source of complete nutrition or may be a source of incomplete nutrition.

As used herein, "complete nutrition" includes nutritional products and compositions that contain sufficient types and levels of macronutrients (protein, fats and carbohydrates) and micronutrients to be sufficient to be a sole source of nutrition for the animal to which it is being administered to. Patients can receive 100% of their nutritional requirements from such complete nutritional compositions.

As used herein, "incomplete nutrition" includes nutritional products or compositions that do not contain sufficient levels of macronutrients (protein, fats and carbohydrates) or micronutrients to be sufficient to be a sole source of nutrition for the animal to which it is being administered to. Partial or incomplete nutritional compositions can be used as a nutritional supplement.

The nutritional compositions of the invention may be a medical food. A medical food is a special class of nutritional composition designed to provide dietary management of certain conditions. The medical food meets certain criteria as set out by and regulated under the Orphan Drug Act of 1983 in Section 5 [360ee](b)(2)(D).

### Nutritional composition ingredients

### Protein source

In an embodiment, the compositions for use according to the invention include a source of protein. The protein source may be dietary protein including, but not limited to animal protein (such as milk protein, meat protein or egg protein), vegetable protein (such as soy protein, wheat protein, rice protein, and pea protein), or combinations thereof. In an embodiment, the protein is selected from the group consisting of whey, chicken, corn, caseinate, wheat, flax, soy, carob, pea or combinations thereof.

### Carbohydrate source

In an embodiment, the compositions include a source of carbohydrates. Any suitable carbohydrate may be used in the present compositions including, but not limited to, starch, sucrose, lactose, glucose, fructose, corn syrup solids, maltodextrin, modified starch, amylose starch, tapioca starch, corn starch, xylitol, sorbitol or combinations thereof.

### Fat source

In an embodiment, the compositions include a source of fat. The source of fat may include any suitable fat or fat mixture. For example, the fat source may include, but is not limited to, vegetable fat (such as olive oil, corn oil, sunflower oil, high-oleic sunflower, rapeseed oil, canola oil, hazelnut oil, soy oil, palm oil, coconut oil, blackcurrant seed oil, borage oil, lecithins, and the like), animal fats (such as milk fat), or combinations thereof. The source of fat may also be less refined versions of the fats listed above (e.g., olive oil for polyphenol content).

### Flavourings etc.

In addition, compositions for use according to the invention may also comprise natural or artificial flavours, for example fruit flavours like banana, orange, peach, pineapple or raspberry or other plant flavours like vanilla, cocoa, coffee, etc.

### Nutritional composition formats

The nutritional compositions may include, besides the main bioactive components and any further bioactive components, and optionally one or more of a protein, carbohydrate and fat source, any number of optional additional food ingredients, including conventional food additives (synthetic or natural), for example one or more acidulants, additional thickeners, buffers or agents for pH adjustment, chelating agents, colorants, emulsifiers, excipient, flavor agent, mineral, osmotic agents, a pharmaceutically acceptable carrier, preservatives, stabilizers, sugar, sweeteners, texturizers, and/or vitamins. The optional ingredients can be added in any suitable amount.

The nutritional composition may be provided in any suitable format.

Examples of nutritional composition formats in which the composition for use according to the invention may be provided include, , solutions, ready-for-consumption compositions (e.g. ready-to-drink compositions or instant drinks), liquid comestibles, soft drinks, juice, sports drinks, milk drinks, milkshakes, yogurt drinks, soup, etc.

In a other embodiments, the nutritional compositions may be provided in the form of a concentrate, a powder, or granules (e.g. effervescent granules), which are diluted with water or other liquid, such as milk or fruit juice, to yield the ready-for-consumption composition.

Further nutritional composition formats include, baked products, dairy products, desserts, confectionery products, cereal bars, and breakfast cereals. Examples of dairy products include milk and milk drinks, yoghurts and other cultured milk products, ice creams and cheeses. Examples of baked products include bread, biscuits and cakes.

In one embodiment, the composition for use according to the invention may also be available in a great variety of formats designed as animal foods, in particular for the dog or the cat, whether in a wet form, semi-wet form or dry form, in particular in the form of biscuits.

### Routes of administration

The nutritional compositions of the present disclosure may be administered by any means suitable for human administration, and in particular for administration in any part of the gastrointestinal tract. Enteral administration, oral administration, and administration through a tube or catheter are all covered by the present disclosure. The nutritional compositions may also be administered by means selected from oral, rectal, sublingual, sublabial, buccal, topical, etc.

The nutritional compositions may be administered in any known form including, for example, tablets, capsules, liquids, chewables, soft gels, sachets, powders, syrups, liquid suspensions, emulsions and solutions in convenient dosage forms. In soft capsules, the active ingredients are preferably dissolved or suspended in suitable liquids, such as fatty oils, paraffin oil or liquid polyethylene glycols. Optionally, stabilizers may be added.

If the nutritional compositions are administered by tube feeding, the nutritional compositions may be used for short term or long term tube feeding.

### Inhibit or decrease cartilage breakdown- in pathologies

The composition of the invention has been shown to decrease the breakdown of cartilage, in particular in joint cartilage.

Joint cartilage is present in joints, for example in finger, knee, hip, jaw, elbow joints.

Thus, the invention in one embodiment relates to a composition according to the invention for use to inhibit or decrease cartilage breakdown, wherein the cartilage breakdown takes place in the context of a pathology with no inflammatory component. In other words, the invention in one embodiment relates to a composition according to the invention for use to inhibit or decrease cartilage breakdown.

Cartilage breakdown may be a result of pathology (either chronic or acute), trauma, or combinations thereof.

Cartilage breakdown takes place both in pathologies dominated by inflammation (such as rheumatoid arthritis) as well as in pathologies where inflammation is not as prominent (eg osteoarthritis). The current use relates breakdown that takes place in the context of a pathology with no inflammatory component.

Trauma can also result in cartilage breakdown processes being initiated. For example, tearing a ligament in the knee leads to destabilization of the knee joint, and breakdown processes will be initiated.

Trauma in the context of the present application refers to a physiological injury caused by an external source, such as for example by falling, or being impacted by a car etc. It also includes what may be called "wear and tear" on the joints.

While often it is preferred to treat trauma with surgery, in one embodiment the present invention relates to a composition of the invention for use in treatment where trauma is treated by surgery and also by administering a composition of the invention.

Thus, embodiments of the use according to the invention include use to inhibit or decrease cartilage breakdown, wherein the cartilage breakdown is a result of a pathology or of trauma.

Examples of pathologies involving cartilage breakdown, and where the compositions of the invention may therefore be useful, include Osteoarthritis, Rheumatoid arthritis, Gout and pseudo-gout, Septic arthritis, Ankylosing spondylitis, Juvenile idiopathic arthritis, Still's disease, Psoriasis (Psoriatic arthritis), Reactive arthritis, Ehlers-Danlos Syndrome, Haemochromatosis, Hepatitis, Lyme disease, Inflammatory bowel disease (Including Crohn's Disease and Ulcerative Colitis), Henoch-Schönlein purpura, Hyperimmunoglobulinemia D with recurrent fever, Sarcoidosis, TNF receptor associated periodic syndrome, Wegener's granulomatosis (and many other vasculitis syndromes), Familial Mediterranean fever, Systemic lupus erythematosus.

In a preferred embodiment, the composition of the invention is for use to inhibit or decrease cartilage breakdown in OA.

### Inhibit or decrease breakdown in non-inflammatory pathologies and trauma

It has been shown herein that compositions of the invention can prevent cartilage breakdown.

Without wishing to be bound by theory it has been observed that while inflammation often leads to cartilage breakdown in joints, cartilage breakdown does also occur in situations where the inflammatory component is much less prounced, and perhaps even negligible.

For example, in OA, the inflammation is secondary to the breakdown of cartilage, i.e. it is a consequence or symptom of the disease, not a cause or driving factor initiating the disease.

For example, trauma to a joint may well initiate cartilage breakdown, without the prominent inflammatory component present for example in RA. Trauma may for example involve tearing ligaments, or impact trauma to a joint for example the knee, fingers. Trauma may also be the accumulation of small insults over time, so called "wear and tear".

In another example, OA is primarily a joint degenerative disease, with a lesser inflammatory component.

It has been shown herein that oleuropein, besides having an anti-inflammatory effect, also can prevent cartilage breakdown, and collagen breakdown.

Thus, the invention relates to a composition for use according to the invention to inhibit or decrease cartilage breakdown, and wherein the cartilage breakdown takes place in the context of a pathology with no inflammatory component, such as trauma or for example OA.

In a preferred embodiment, the invention relates to a composition for use according to the invention wherein the use is to inhibit or decrease cartilage breakdown in OA.

In further embodiments, the invention relates to oleuropein for use to inhibit or decrease cartilage breakdown, where the cartilage breakdown takes place in the context of a pathology with no inflammatory component, such as trauma or for example OA.

### Inhibit or decrease collagen breakdown

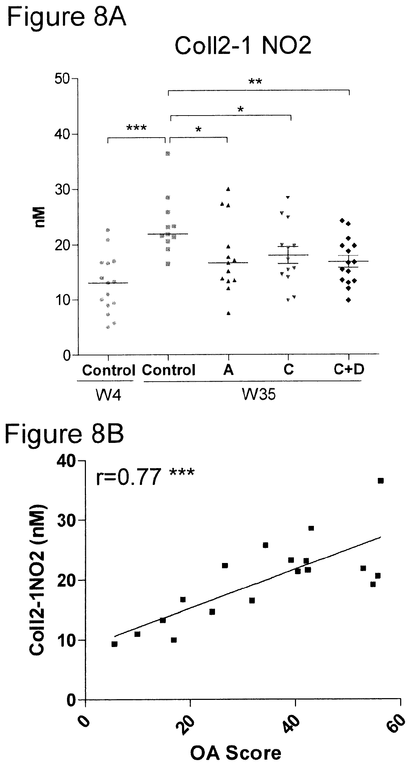

Oleuropein is shown herein to decrease appearance of breakdown products of collagen (e.g. Figure 8A). Thus oleuropein acts to inhibit or decrease collagen breakdown in cartilage.

Thus, the invention in one embodiment relates to the use of oleuropein to inhibit or decrease cartilage breakdown. In a further embodiment, the invention relates to the use of oleuropein to inhibit or decrease collagen breakdown, in particular in cartilage. Further embodiments relate to the use of oleuropein to inhibit or decrease collagen II breakdown, in particular in cartilage.

Other embodiments relate to a composition for use according to the invention as described herein elsewhere, to inhibit or decrease cartilage breakdown. In another embodiment of the invention relates to the composition for use according to the invention, wherein the use is to inhibit or decrease breakdown of collagen in cartilage, for example breakdown of collagen II in cartilage.

Collagen II breakdown is an important event in cartilage breakdown. Without wishing to be bound by theory, it is thought that release of glucosaminoglycans from articular cartilage is an event which may be reversible. However, the breakdown of collagen means that the fibril network is compromised. The breakdown of collagen is a later event than glycosaminoglycan loss, and may be less reversible than glycosaminoglycan loss is. This means that it is very important to inhibit or decrease the breakdown of collagen in the joint, especially collagen II.

The inhibiting and/or decreasing of cartilage breakdown includes inhibiting and/or decreasing cartilage breakdown, and also includes inhibiting and/or decreasing collagen breakdown. The main collagen in cartilage is collagen II, thus inhibiting or decreasing collagen breakdown includes inhibiting and/or decreasing collagen II breakdown.

It has been shown that the composition of the invention can inhibit proteases that are known to be active in cartilage breakdown. Inhibiting or decreasing the proteolytic activity will inhibit or decrease the breakdown of the components of cartilage. It is especially important to inhibit or decrease the breakdown of the collagen fibril network, as this network is important in order to retain the structural integrity of the cartilage.

Thus, in one embodiment, the invention relates to a composition for use according to the invention wherein the use is to inhibit or decrease breakdown of collagen in cartilage. In further embodiments, the invention relates to a composition for use according to the invention wherein the use is to inhibit or decrease breakdown of collagen II in cartilage.

### Treat or prevent decreased mobility with age

The compositions for use according to the invention have been shown to inhibit or decrease proteolytic activity.

Ageing leads to cartilage breakdown. The guinea pig model used shows that cartilage breakdown appears spontaneously with age.

Thus, the invention relates to a composition of the invention for use to inhibit or decrease cartilage breakdown associated with ageing, wherein the cartilage breakdown takes place in the context of a pathology with no inflammatory component.

In another embodiment, the invention relates to a composition of the invention for use to inhibit or decrease collagen breakdown in cartilage breakdown associated with ageing, wherein the cartilage breakdown takes place in the context of a pathology with no inflammatory component, for example use to inhibit or prevent collagen II breakdown in cartilage associated with ageing.

The breakdown of cartilage can contribute to joint stiffness and joint pain, leading to decreased mobility in the patient.

The compositions for use according to the invention may in other embodiments be used for maintaining or improving joint functionality, including cartilage functionality, during ageing.

In a further embodiment the invention relates to a composition for use according to the invention to improve mobility in a subject, for example in adult or elderly mammals.

Thus, in a preferred embodiment the composition according the invention may be for use to improve activity and/or mobility of the individual, for example by preventing osteoarthritis, and/or by inhibiting or decreasing cartilage breakdown, wherein the cartilage breakdown takes place in the context of a pathology with no inflammatory component.

Other preferred embodiments relate to a composition for use according to the invention wherein the use is to prevent cartilage breakdown, wherein the cartilage breakdown takes place in the context of a pathology with no inflammatory component, and thus maintain healthy joints, or to maintain or improve mobility.

In a further embodiment the compositions of the invention may be for use to maintain the status of the cartilage.

### Specific combinations oleuropein+ curcumin+ quercetin

The composition according to the invention which comprises oleuropein, curcumin and quercetin strongly downregulates proteases associated with cartilage breakdown.

Especially in situations mimicking disease, this combination is highly effective at downreguating proteases associated with cartilage breakdown.

Thus, the invention in one embodiment relates to this composition for use to decrease or inhibit cartilage breakdown, wherein the cartilage breakdown takes place in the context of a pathology with no inflammatory component.

For example, this composition may be useful in preventing OA.

### Inflammation and cartilage breakdown

Compositions according to the invention which comprise oleuropein and further at least one of curcumin and quercetin, have been shown to both downregulate proteases which are active in cartilage breakdown, as well as downregulate an inflammation marker.

Thus, the invention in another embodiment relates to these compositions for use to inhibit or decrease cartilage breakdown, wherein the cartilage breakdown takes place in the context of a pathology with no inflammatory component, as well as to inhibit inflammation.

The composition according to the invention which comprises oleuropein, curcumin and quercetin strongly downregulates proteases associated with cartilage breakdown, and furthermore downregulates an inflammation marker.

Thus, the invention in one embodiment relates to this composition for use to decrease or inhibit cartilage breakdown, wherein the cartilage breakdown takes place in the context of a pathology with no inflammatory component, and inhibits inflammation.

For example, these compositions may be useful in prevention of RA.

### Joint disease

In a further embodiment the invention relates to compositions for use according to the invention, wherein the composition comprises oleuropein and further comprises at least one selected from the group consisting of curcumin and quercetin.

These compositions of the invention are shown to downregulate proteolytic enzymes associated with cartilage breakdown, and also to have a downregulating effect on COX2, which is an inflammatory marker.

Thus, one embodiment of the invention relates to these said compositions of the invention for use in preventing joint disease. Said joint disease may be any joint disease, such as for example degenerative joint disease, for example OA.

### Target groups

Target groups for the composition for use according to the invention may be any mammal displaying cartilage breakdown, for example because they suffer from one or more of the pathologies involving cartilage breakdown mentioned herein. Cartilage breakdown may be detected by visual means, such as by radiography. Alternatively, the detection of breakdown products of cartilage may detected in bodily fluid. For example one or more collagen II epitopes such as (Col2-1, Col2-1 NO, CTX-II) may be detected in for example samples, such as plasma or urine samples.

Another target group may be any mammal who does not yet display cartilage breakdown, but who are at risk for cartilage breakdown, for example at risk for OA or any of the pathologies involving cartilage breakdown mentioned herein.

A particular embodiment of the invention relates to the composition for use to improve activity and/or mobility of the individual, for example by preventing osteoarthritis, and/or by inhibiting or decreasing cartilage breakdown in elderly or aging individuals, wherein the cartilage breakdown takes place in the context of a pathology with no inflammatory component.

In further embodiments, the composition for use according to the invention may be for use in mammals, such as humans, or pets. Examples of pets include cats, dogs, and horses.

Though the invention may be useful in many various age groups, in a preferred embodiment the compositions for use to increase mobility according to the invention are targeted to ageing population, in particular healthy aging and/or elderly mammals.

### Method of manufacturing a nutritional composition of the invention

The nutritional composition for use according to the invention, may be manufactured by a method comprising the step of:
- providing ingredients for a nutritional composition and oleuropein and mixing, such that the nutritional composition comprises oleuropein.

A nutritional composition for use according to the invention may be manufactured by a method comprising the step of
- providing one or more ingredients for a nutritional composition, oleuropein and/or hydroxytyrosol, and optionally further one or more of curcumin and quercetin, and mixing.

### Pharmaceutical composition for use.

In a further embodiment, the invention relates to a composition for use to inhibit or prevent cartilage breakdown according to the invention, wherein the cartilage breakdown takes place in the context of a pathology with no inflammatory component, wherein the composition is a pharmaceutical composition.

By pharmaceutical means a composition, other than a nutritional composition, wherein a substance is used on or in the body to prevent, diagnose, alleviate, treat, or cure a disease in humans or animals in medicine. According to the present invention, the pharmaceutical may be used for inhibiting or decreasing cartilage breakdown, wherein the cartilage breakdown takes place in the context of a pathology with no inflammatory component.

The pharmaceutical may be for use by a human. It may alternatively be a veterinary composition, for example suited for a dog, cat, or horse, in particular a thoroughbred horse.

The pharmaceutical composition of the invention comprises oleuropein.

The invention further relates to uses of the pharmaceutical according to the invention, as described herein as use of the compositions of the invention.

A pharmaceutical composition for use according to the invention comprising oleuropein, or further comprising curcumin and/or quercetin, in combination with at least one excipient selected from the group constituted by the pharmaceutically acceptable excipients. Procedures for the preparation of pharmaceutical compositions according to the invention can easily be found by the specialist skilled in the art, for example in the handbook Remington's Pharmaceutical Sciences, Mid. Publishing Co, Easton, Pa., USA. Physiologically acceptable excipients, vehicles and adjuvants are also described in the handbook entitled "Handbook of Pharmaceutical Excipients, Second edition, American Pharmaceutical Association, 1994. In order to formulate a pharmaceutical composition according to the invention, the specialist skilled in the art will advantageously be able to refer to the latest edition of the European Pharmacopoeia or the Pharmacopoeia of the United States of America (USP). The specialist skilled in the art will in particular be able advantageously to refer to the fourth edition "2002" of the European Pharmacopoeia or also to the edition USP 25-NF 20 of the American Pharmacopoeia (U.S. Pharmacopoeia).

Advantageously, a pharmaceutical composition such as defined above is suitable for oral, parenteral or intravenous administration. When the pharmaceutical composition for use according to the invention comprises at least one pharmaceutically or physiologically acceptable excipient, it is in particular an excipient appropriate for administration of the composition by the oral route or an excipient suitable for administration of the composition by the parenteral route.

A pharmaceutical composition for use according to the invention is available indifferently in a solid or liquid form. For oral administration, a solid pharmaceutical composition in the form of tablets, capsules or gelatine capsules will be preferred.

In liquid form, a pharmaceutical composition in the form of an aqueous or non-aqueous suspension, or also in the form of a water-in-oil or oil-in-water emulsion will be preferred.

Solid pharmaceutical forms may comprise, as vehicles, adjuvants or excipients, at least one diluent, one flavour, one solubilising agent, one lubricant, one suspension agent, one binder, one disintegrating agent and one encapsulating agent. Such compounds are for example magnesium carbonate, magnesium stearate, talc, lactose, pectin, dextrin, starch, gelatine, cellulosic materials, cocoa butter, etc. The compositions in liquid form may also comprise water, possibly as a mixture with propylene glycol or polyethylene glycol, and possibly also colouring agents, flavours, stabilisers and thickening agents.

### Use in methods of treatment

Also described herein is a composition for use in a method of inhibiting or decreasing of cartilage breakdown, wherein the cartilage breakdown takes place in the context of a pathology with no inflammatory component, for example a pathology in which cartilage breakdown takes place or a trauma which is associated with cartilage breakdown, said use comprising administering to an individual in need thereof an effective amount of a composition according to the invention. For example, the use comprises administering an effective amount of oleuropein. The use in a method of treatment comprises administering an effective amount of the composition for use according to the invention comprising oleuropein, as well as one or more further polyphenol, for example one or more further polyphenols selected from the group consisting of curcumin, quercetin and rutin.

As used herein, "effective amount" is an amount that prevents a deficiency, treats a disease or medical condition in an individual or, more generally, reduces symptoms, manages progression of the diseases or provides a nutritional, physiological, or medical benefit to the individual.

The effective amount of a composition which is required to achieve a therapeutical effect will, of course, vary with the particular composition, the route of administration, the age and condition of the recipient, and the particular disorder or disease being treated.

Also described is a composition for use in methods of preventing or treating pathologies involving cartilage breakdown, wherein the cartilage breakdown takes place in the context of a pathology with no inflammatory component, such as for example OA; inhibiting or decreasing cartilage breakdown; inhibiting or decreasing collagen breakdown in cartilage; inhibiting or decreasing collagen II breakdown in cartilage; which methods comprise administering an effective amount of a composition for use according to the invention to an individual.

The use in a method of treatment may concern preventing or treating osteoarthritis.

The use in methods of treatment may be in a mammal, such as a human, or a pet, for example a dog, a cat and/or a horse.

The composition for use in the method of treatment, may be administered as one or more nutritional compositions of the invention and/or pharmaceutical compositions of the invention.

### Combination of disclosures

It should be noted that embodiments and features described in the context of one of the aspects of the present invention also apply to the other aspects of the invention.

The compositions for use according to the invention are herein described in different parameters, such as the ingredients, nutritional composition formats, uses, target groups etc. It should be noted that embodiments and features described in the context of one of the parameters of the composition for use according to the invention, may also be combined with other embodiments and features described in the context of another parameter, unless expressly stated otherwise.

The invention will now be described in further details in the following non-limiting examples.

### Examples

### Example 1 - In vitro study - bovine primary chondrocytes in alginate beads

### Materials and methods

Oleuropein. Substantially purified oleuropein (>98%) was purchased from Extrasynthese (Genay, France).

Hydroxytyrosol. Substantially purified hydroxytyrosol (>98%) was purchased from Extrasynthese (Genay, France). Metabolite of oleuropein.

Quercetin. Substantially purified quercetin (>97%) was purchased from HWI Analytik GmbH (Ruelzheim, Germany). Metabolite of rutin.

Rutin. Substantially purified rutin (>94%) was purchased from Sigma-Aldrich (Buchs, Switzerland).

Curcumin.Substantially purified rutin (>70%) was purchased from Sigma-Aldrich (Buchs, Switzerland).

### Cellular culture

The foot of a young cow was washed to remove soil. The skin was removed from the foot. The articulation was opened transversally. Intra articular ligaments were transected. The opened articulation was washed with phosphate buffered saline. With a scalpel blade, full thickness slices of cartilage were dissected out and collected in DMEM high glucose with 10mM hepes and 1% Penicillin-Streptomycin. Slices of cartilage were washed in DMEM high glucose with 10mM hepes and 1% Penicillin-Streptomycin.

Cartilage was then digested successively by 0.5 mg/ml of hyluronidase during 30 minutes, 1.0 mg/ml of pronase E during 60 minutes and 1.0 mg/ml of collagenase overnight under slow agitation.

After the enzymatic incubation, the cell suspension was filtered through a 70 µm cell strainer to separate cell cluster and remove tissue debris. The cell suspension was centrifuged to pellet the cells.

The media was discarded and the cell pellet was washed by centrifugation and resuspended in a 0.9% sodium chloride solution containing 10 mM hepes.

Cells were suspended in a solution of 0.9% NaCl, 10 mM HEPES containing 1.2% alginate to obtain a density of 4.3×10⁶ cells per ml. The suspension was passed through a needle in a drop-wise fashion into a 10²mM CaCl₂ solution. After 10 min, the beads were polymerized and were washed with 0.9% NaCl, 10 mM HEPES. 10 beads were deposited in each well of a 24 wells culture plate with 1 ml of complete medium (DMEM high glucose with 2mM L-glutamine, 0.5 mg/ml ascorbic acid, 0.2 mg/ml L-proline and 10% fetal calf serum). Cells were maintained in the medium for 2 days and then treatments of 1.5 µM of oleuropein, 1.5 µM of hydroxytyrosol, 1.5 µM of quercetin, 1.5 µM of curcumin, 1.5 µM of oleuropein + 1.5 µM of quercetin, 1.5 µM of oleuropein + 1.5 µM of curcumin, 1.5 µM of hydroxytyrosol + 1.5 µM of quercetin, 1.5 µM of hydroxytyrosol + 1.5 µM of curcumin, 1.5µM of quercetin + 1.5µM of curcumin, 1.5 µM of oleuropein + 1.5µM of quercetin + 1.5µM of curcumin, 1.5 µM of hydroxytyrosol + 1.5 µM of quercetin + 1.5 µM of curcumin in presence or in absence of interleukin -1 alpha. Treatment were dissolved in dimethylslfoxide (DMSO). Final concentration of DMSO was 0.1%.The cells were exposed to treatments for 3 days.

At the end of the treatments, beads were washed and resuspended in 0.1M sodium citrate + 0.15 M sodium chloride solution. Suspension was centrifuged and pellet containing cells was collected for total RNA extraction.

### Gene expression

Total RNA was isolated from pool of 40 beads and isolated using RNeasy kit from Qiagen. cDNA was obtained by reverse transcription of RNA with the Primescript 1st strand cDNA Synthesis kit from Takara. mRNA expression of ADAMTS-5, COX-2, MMP-13 and GAPDH were quantified in real-time PCR (SYBR Green)

Primers (from Bos Taurus) are listed below in Table 1:

**Table 1 Primers**

| Gene | Forward primer | Reverse primer |
|---|---|---|
| GAPDH | AAG GGC ATT CTA GGC TAC ACT GA (SEQ ID NO. 1) | AGA GTG AGT GTC GCT GTT GAA GTC (SEQ ID NO. 2) |
| ADAMTS-5 | CAC CTC AGC CAC CAT CAC AG (SEQ ID NO. 3) | AGT ACT CTG GCC CGA AGG TC (SEQ ID NO. 4) |
| COX-2 | CAC CCA TCA ATT TTT CAA GAC AGA (SEQ ID NO. 5) | CCA CCC CAT GGT TCT TTC C (SEQ ID NO. 6) |
| MMP-13 | GCA GAG AGC TAC CTG AAA TCA TAC TAC T (SEQ ID NO. 7) | AAT CAC AGA GCT TGC TGC AGT TT (SEQ ID NO. 8) |

### Results

Figures 1 to 3 show mRNA expression of ADAMTS-5, COX-2 and MMP-13 normalized by GAPDH mRNA expression after 3 days of culture. Data were expressed as fold change in gene expression compared to negative control (cells cultured in complete medium). Results were calculated according to the 2 ^{-ΔΔCT} method.

| | |
|---|---|
| CTL- | negative control |
| OLP | oleuropein |
| HTY | hydroxytyrosol |
| QRC | quercetin |
| CUR | curcumin |
| IL1a | interleukin-1 alpha 10 ng/ml |

Data were expressed as median ± S.E.Median (2 experiments of 3 replicates each). Statistics were done by Kruskal Wallis test followed by Mann Whitney U test (2 tailed) *p<0.05; **<0.01; ***p<0.001 vs negative control (CTL-) or interleukin-1 alpha (IL1a).

Figure 1A shows ADAMTS-5 mRNA expressions relative to GAPDH in absence of ILia which mimics a normal state of the cells. Conditions were statistically compared to CTL-.

Figures 1B shows ADAMTS-5 mRNA expressions relative to GAPDH in presence of ILia which mimics a disease state of the cells. Conditions were statistically compared to IL1a except IL1a which was compared to CTL- (validation of the experiment).

In normal conditions, additions of OLP, HTY, QRC or CUR decrease expression of ADAMTS-5 (marker of cartilage destruction). Combinations of these different polyphenols have a synergistic effect.

In disease conditions, additions of OLP, HTY, QRC or CUR can counteract IL-1 alpha stimulated chondrocytes and decrease expression of ADAMTS-5. Combinations of these different polyphenols have a synergistic effect.

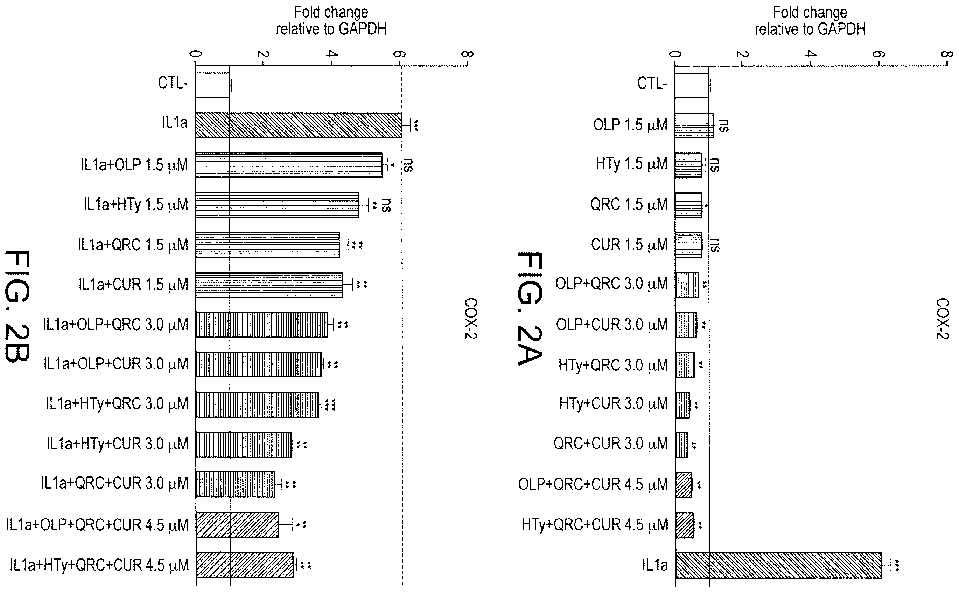

Figure 2A shows COX-2 mRNA expressions relative to GAPDH in absence of ILia which mimics a normal state of the cells. Conditions were statistically compared to CTL-.

Figures 2B shows COX-2 mRNA expressions relative to GAPDH in presence of ILia which mimics a disease state of the cells. Conditions were statistically compared to IL1a except IL1a which was compared to CTL- (validation of the experiment).

In normal conditions, addition of QRC can reduce expression of COX-2 (marker of inflammatory status). Combinations of OLP, HTY, QRC or CUR by two or three have a synergistic effect.

In disease conditions, additions QRC or CUR can counteract IL-1 alpha stimulated cells and decrease expression of COX-2. Combination of OLP, HTY, QRC or CUR by two or three have a synergistic effect.

Figure 3A shows MMP-13 mRNA expressions relative to GAPDH in absence of ILia which mimics a normal state of the cells. Conditions were statistically compared to CTL-.

Figures 3B shows MMP-13 mRNA expressions relative to GAPDH in presence of ILia which mimics a disease state of the cells. Conditions were statistically compared to IL1a except IL1a which was compared to CTL- (validation of the experiment).

In normal conditions, additions of OLP, HTY, QRC or CUR alone or in combination decrease expression of MMP-13 (involved in the breakdown of extracellular matrix, linked to articular cartilage turnover in OA).

In disease conditions, additions of OLP, HTY, QRC or CUR can counteract IL-1 alpha stimulated chondrocytes and decrease expression of MMP-13. Combinations of OLP, HTY, QRC or CUR by three have a synergistic effect.

### Example 2 In vivo study - spontaneous development of osteoarthritis in Hartley guinea pigs

### Animals & treatments

Sixty-five male Hartley guinea-pigs aged 3 weeks were obtained from Charles River Laboratories (Paris). Animals were housed 5 per cage in solid bottom cages and fed with standard guinea-pig diet containing Vitamin C (1 mg/g) and Vitamin D3 (3.4 IU/g), as well as water ad libitum. All animals were allowed one week for acclimatization to housing conditions prior to oleuropein, rutin and/or curcumin administration. PVC pipes were added to the cages to improve housing conditions and minimize stress.

After one week, male Hartley guinea-pigs aged 4-weeks were randomized in experimental groups:
- Group A (n=15) received a daily dose of oleuropein during 31-weeks
- Group B (n=15) received standard diet during 31-weeks (Control);
- Group C (n=15) received a daily dose of rutin during 31-weeks
- Group D (n=15) received a daily dose of rutin and curcumin during 31-weeks
- Group E (n=5, Reference group) were euthanized at inclusion and didn't receive any intervention.

Oleuropein, rutin and curcumin were integrated in the diet. Animals were weighted each week and identification was made by microchip. Food intake was controlled at W9, W15, W21, W26 and W34 by weighing daily quantities of food added and remaining.

### Blood collection

6h fasting blood was obtained in the morning, under ketamine/xylazine tranquilization, at the superficial veins at the ears, each 6 weeks: W4 (=T0), W10, W16, W22, W28. At W35, blood was collected by intracardiac puncture just before euthanasia, under general anaesthesia.

### Euthanasia

After weighing, animal euthanasia was performed after intracardiac blood puncture under sodium pentobarbital 100mg/kg. Mains vital organs were observed to detect anomalies (heart, digestive and urinary tracts, adrenal glands). Liver and adrenal glands were weighed. The right knee joint from each animal will be fixed for 24 h in 4% buffered paraformaldehyde, followed by decalcification in HCl acid (DC2, Labonord, Belgium) for 4h at 4°C before paraffin embedding.

### Histology

Paraffin embedded right knees were cut with a microtome (Leica) in 6 µm sections, in the central area not covered by meniscus, following the Cushin plane, as recommended by OARSI.
3 sections at 200 µm interval were stained with hematoxylin, fast green and safranin-O, and 1 supplementary central section was stained with toluidine blue.

Each compartment of the section (tibial median, tibial lateral, femoral median and femoral lateral) was scored by 2 blinded trained experts following OARSI recommendation and to assess the global score, each compartment score was added.

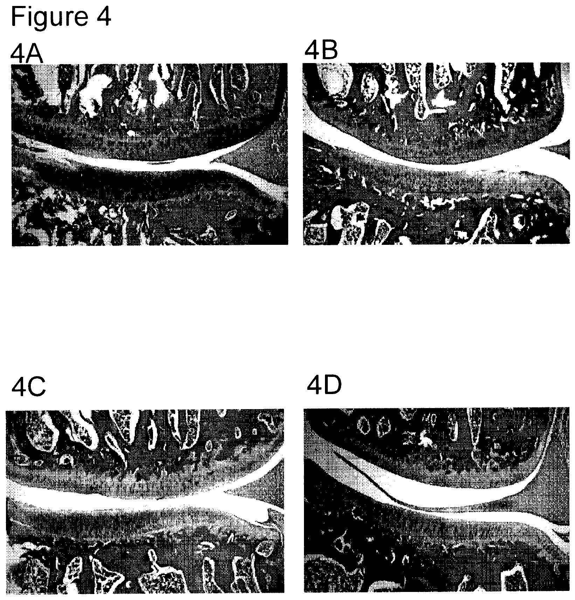

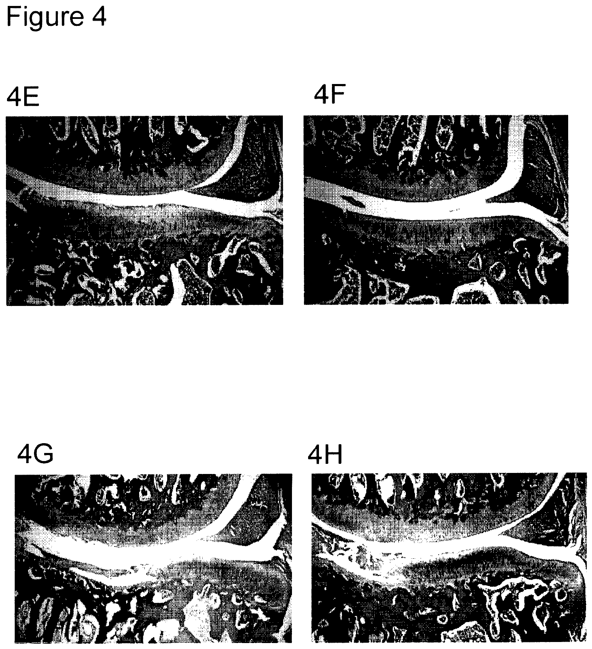

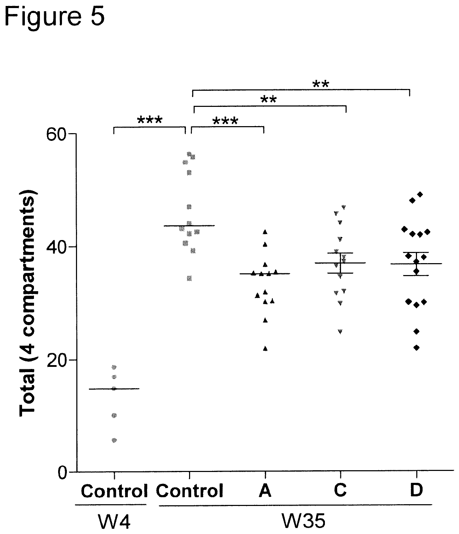

Results are shown in Figures 4 and 5.

### PGE2, Coll2-1NO2 and Fib3-1 assays

PGE2 was measured in guinea pig serum using a competitive ELISA kit (Arbor Assays, USA).

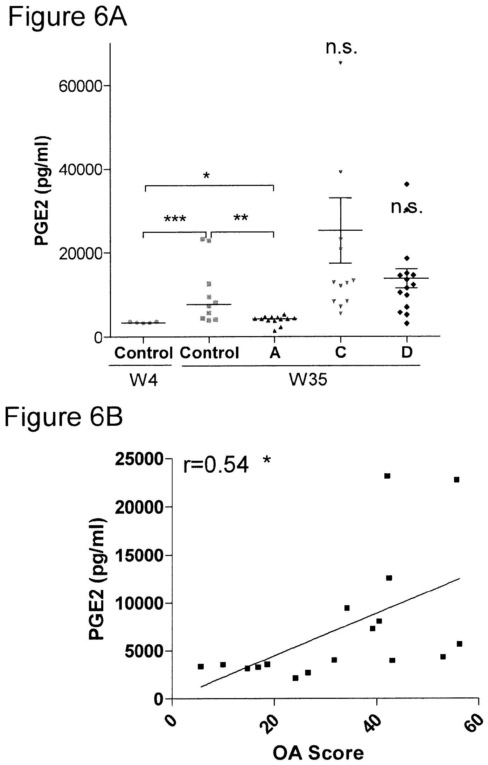

Results are shown in Figure 6.Oleuropein treatment can decrease PGE2 levels in plasma after 35 weeks of treatments.

PGE2 level in plasma can be positively correlated to OA score. This confirms that PGE2 is a relevant biomarker to OA onset and progression in guinea pig model and to evaluate efficacy of treatments.The nitrosylated epitope, Coll2-1NO2, localized to the helical domain of type II collagen was quantified in guinea-pig sera by competitive ELISA in triplicate with polyclonal rabbit antisera (D37, Artialis, Belgium). The Coll2-1NO2 immunoassay quantified with a high specificity and affinity the nitrated amino acids sequence.

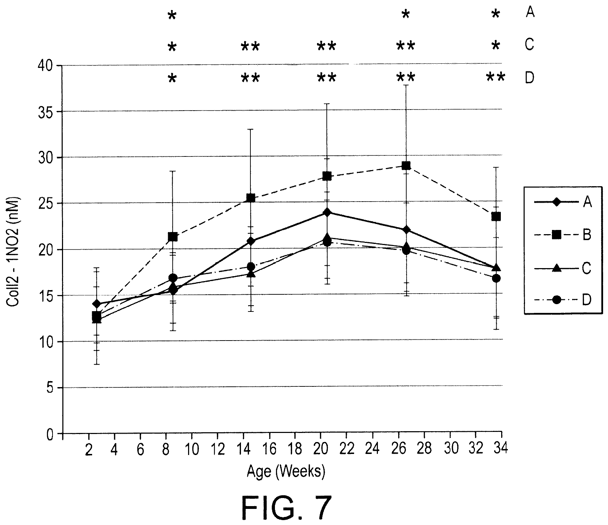

Results are shown in Figures 7 and 8. Oleuropein, rutin and rutin + curcumin treatments can decrease collagen breakdown measured by levels of coll2-1 NO2 in plasma. Amount of coll2-1 NO2 is positively correlated to the OA score.

This confirms that Coll-2-1 NO2 is a relevant biomarker to up OA onset and progression in guinea pig model and to evaluate efficacy of treatments.

Fib3-1 is fragments of fibulin-3 increased in sera of OA patients. Fib3-1 was quantified in guinea pig sera by competitive ELISA in triplicate with polyclonal rabbit antisera (AS88, Artialis, Belgium).

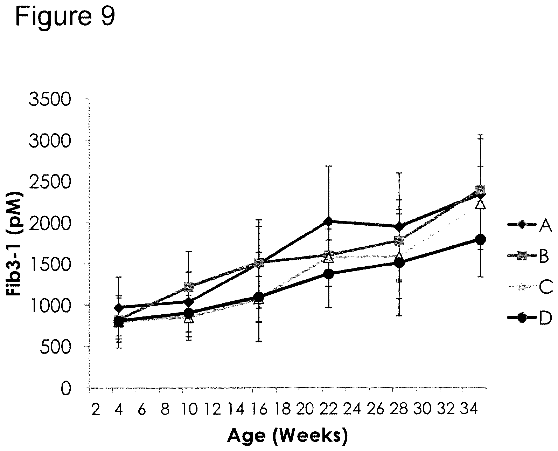

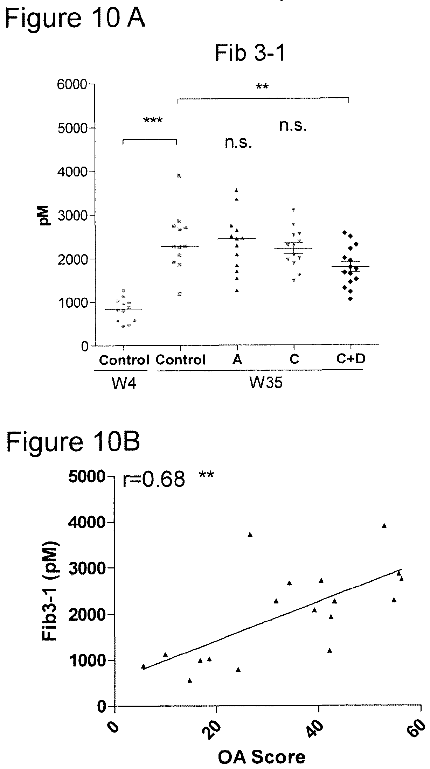

Results are shown in Figures 9 and 10. Rutin + curcumin treatment can decrease fibulin 3-1 levels in plasma. Fibulin 3-1 level in plasma can be positively correlated to OA score. This confirms that fib 3-1 is a relevant biomarker to OA onset and progression in guinea pig model and to evaluate efficacy of treatments.

The intra- and inter-assays CVs were lower than 10% and the dilution curves were parallel to the standard curve for both assays.

### Results

The results (mean ± SD) were calculated for each parameter. Following a normality test, a parametric ANOVA with Dunnett's post-test or Pearson correlation was performed on all the experiments. *p<0.05; **<0.01; ***p<0.001.

Control W4 Group E (n=5, Reference group) animals were euthanized at inclusion and didn't receive any intervention.

| | |
|---|---|
| Control W35 | Group B (n=15) received standard diet during 31-weeks. |
| A W35 | Group A (n=15) received a daily dose of oleuropein during 31-weeks. |
| C W35 | Group C (n=15) received a daily dose of rutin during 31-weeks. |
| D W35 | Group D (n=15) received a daily dose of rutin and curcumin during 31-weeks. |

Oleuropein, rutin and rutin + curcumin treatments can decrease OA score in guinea pigs by different mechanisms.

## Claims

1. Composition comprising oleuropein for use to inhibit or decrease cartilage breakdown, wherein the cartilage breakdown takes place in the context of a pathology with no inflammatory component.

2. Composition for use according to any of the preceding claims, wherein the composition further comprises curcumin.

3. Composition for use according to any of the preceding claims, wherein the composition further comprises quercetin.

4. Composition for use according to any of the preceding claims wherein the composition further comprises at least one compound selected from the group consisting of antioxidants, anti-inflammatory compounds, glycosaminoglycans, prebiotics, fibres, probiotics, fatty acids, minerals, trace elements and/or vitamins.

5. Composition for use according to claim 4 wherein the composition further comprises at least one anti-inflammatory compound.

6. Composition for use according to claim 4 to 5, wherein the at least one anti-inflammatory compound is a plant extract.

7. Composition for use according to claim 6 wherein the plant extract is from one or more plants selected from the group consisting of devil's claw (*Harpagophytum procumbens*), *Rosmarinus officinalis,* hyssop, ginger *(Zingiber officinale),* turmeric (*Curcuma longa*), Arnica Montana, willow bark, pomegranate *(Punica granatum),* green tea *(Camellia sinensis),* cat's claw *(Uncaria tometosa* and *Uncaria guianensis),* Indian olibaum (*Boswelia serrata*), and pineapple bromelain (*Ananas comosus*), Black seed (*Nigella sativa*), St. John's wort, hyperforin, Goldenseal, German Chamomile *(Matricaria recutita).*

8. Composition for use according to claim 4 to 7, wherein the composition further comprises at least one antioxidant.

9. Composition for use according to claim 8 wherein the at least one antioxidant is selected from the group consisting of astaxanthin, carotenoids, coenzyme Q10 ("CoQ10"), flavonoids, glutathione, Goji (wolfberry), hesperidin, lactowolfberry, lignan, lutein, lycopene, polyphenols, selenium, vitamin A, vitamin C, vitamin E, zeaxanthin.

10. Composition for use according to any of the preceding claims wherein the composition is a nutritional composition.

11. Composition for use according to claims 1 to 9, wherein the composition is a pharmaceutical composition.

12. Composition for use according to claim 10, wherein said nutritional composition is a medical food.

13. Composition for use according to any of the preceding claims wherein the use is to inhibit or decrease breakdown of collagen in cartilage.

14. Composition for use according to any of the preceding claims wherein the use is to improve activity and/or mobility of an individual.

## Patentansprüche

1. Zusammensetzung, umfassend Oleuropein zur Verwendung, um Knorpelzerfall zu hemmen oder zu verringern, wobei der Knorpelzerfall im Kontext einer Pathologie ohne Entzündungskomponente stattfindet.

2. Zusammensetzung nach einem der vorstehenden Ansprüche, wobei die Zusammensetzung ferner Curcumin umfasst.

3. Zusammensetzung nach einem der vorstehenden Ansprüche, wobei die Zusammensetzung ferner Quercetin umfasst.

4. Zusammensetzung zur Verwendung nach einem der vorstehenden Ansprüche, wobei die Zusammensetzung ferner mindestens eine Verbindung umfasst, die aus der Gruppe ausgewählt ist, bestehend aus Antioxidationsmitteln, entzündungshemmenden Verbindungen, Glykosaminoglykanen, Prebiotika, Fasern, Probiotika, Fettsäuren, Mineralien, Spurenelementen und/oder Vitaminen.

5. Zusammensetzung zur Verwendung nach Anspruch 4, wobei die Zusammensetzung ferner mindestens eine entzündungshemmende Verbindung umfasst.

6. Zusammensetzung zur Verwendung nach Anspruch 4 bis 5, wobei die mindestens eine entzündungshemmende Verbindung ein Pflanzenextrakt ist.

7. Zusammensetzung zur Verwendung nach Anspruch 6, wobei das Pflanzenextrakt aus einer oder mehreren Pflanzen stammt, die aus der Gruppe ausgewählt sind, bestehend aus Teufelskralle (*Harpagophytum procumbens*), *Rosmarinus officinalis,* Ysop, Ingwer *(Zingiber officinale),* Kurkuma (*Curcuma longa*), Echte Arnika, Weidenrinde, Granatapfel *(Punica granatum),* Teepflanze *(Camellia sinensis),* Katzenkralle *(Uncaria tometosa* und *Uncaria guianensis),* Weihrauch (*Boswelia serrata*) und Ananas-Bormelin (*Ananas comosus*), Echter Schwarzkümmel (*Nigella sativa*), Johanniskraut, Hyperforin, Goldsiegelwurzel, Echte Kamille (*Matricaria recutita*)*.*

8. Zusammensetzung zur Verwendung nach Anspruch 4 bis 7, wobei die Zusammensetzung ferner mindestens ein Antioxidationsmittel umfasst.

9. Zusammensetzung zur Verwendung nach Anspruch 8, wobei mindestens ein Antioxidationsmittel aus der Gruppe ausgewählt ist, bestehend aus Astaxanthin, Carotinoiden, Coenzym Q10 ("CoQ10"), Flavonoiden, Glutathion, Goji-Beere (Wolfsbeere), Hesperidin, Lacto-Wolfsbeere, Lignan, Lutein, Lycopin, Polyphenolen, Selen, Vitamin A, Vitamin C, Vitamin E, Zeaxanthin und Kombinationen davon.

10. Zusammensetzung zur Verwendung nach einem der vorstehenden Ansprüche, wobei die Zusammensetzung eine Nährstoffzusammensetzung ist.

11. Zusammensetzung zur Verwendung nach Anspruch 1 bis 9, wobei die Zusammensetzung eine pharmazeutische Zusammensetzung ist.

12. Zusammensetzung zur Verwendung nach Anspruch 10, wobei die Nährstoffzusammensetzung ein medizinisches Lebensmittel ist.

13. Zusammensetzung zur Verwendung nach einem der vorstehenden Ansprüche, wobei die Verwendung dazu dient, Kollagenzerfall in dem Knorpel zu hemmen oder zu verringern.

14. Zusammensetzung zur Verwendung nach einem der vorstehenden Ansprüche, wobei die Verwendung dazu dient, Aktivität und/oder Mobilität eines Individuums zu verbessern.

## Revendications

1. Composition comprenant de l'oleuropéine utilisable pour inhiber ou diminuer une dégradation cartilagineuse, dans laquelle la dégradation cartilagineuse a lieu dans le contexte d'une pathologie dépourvue de composante inflammatoire.

2. Composition pour utilisation selon l'une quelconque des revendications précédentes, dans laquelle la composition comprend en outre de la curcumine.

3. Composition pour utilisation selon l'une quelconque des revendications précédentes, dans laquelle la composition comprend en outre de la quercétine.

4. Composition pour utilisation selon l'une quelconque des revendications précédentes dans laquelle la composition comprend en outre au moins un composé choisi dans le groupe constitué d'antioxydants, composés antiinflammatoires, glucosaminoglycanes, prébiotiques, fibres, probiotiques, acides gras, minéraux, oligoéléments et/ou vitamines.

5. Composition pour utilisation selon la revendication 4 dans laquelle la composition comprend en outre au moins un composé anti-inflammatoire.

6. Composition pour utilisation selon la revendication 4 à 5, dans laquelle l'au moins un composé anti-inflammatoire est un extrait de plante.

7. Composition pour utilisation selon la revendication 6 dans laquelle l'extrait de plante provient d'une ou plusieurs plantes choisies dans le groupe constitué de griffe du diable (*Harpagophytum procumbens), Rosmarinus officinalis,* hysope, gingembre *(Zingiber officinale),* curcuma (*Curcuma longa*)*,* Arnica Montana, écorce de saule, grenade *(Punica granatum),* thé vert *(Camellia sinensis),* griffe de chat *(Uncaria tometosa* et *Uncaria guianensis),* encens indien (*Boswelia serrata*), et bromélaïne d'ananas (*Ananas comosus*), nigelle (*Nigella sativa*), millepertuis, hyperforine, hydraste du Canada, camomille allemande (*Matricaria recutita).*

8. Composition pour utilisation selon la revendication 4 à 7, dans laquelle la composition comprend en outre au moins un antioxydant.

9. Composition pour utilisation selon la revendication 8 dans laquelle l'au moins un antioxydant est choisi dans le groupe constitué d'astaxanthine, caroténoïdes, coenzyme Q10 (« CoQ10 »), flavonoïdes, glutathion, goji (baie de goji), hespéridine, lacto-symphorine occidentale, lignane, lutéine, lycopène, polyphénols, sélénium, vitamine A, vitamine C, vitamine E, zéaxanthine.

10. Composition pour utilisation selon l'une quelconque des revendications précédentes dans laquelle la composition est une composition nutritionnelle.

11. Composition pour utilisation selon les revendications 1 à 9, dans laquelle la composition est une composition pharmaceutique.

12. Composition pour utilisation selon la revendication 10, dans laquelle ladite composition nutritionnelle est un aliment médical.

13. Composition pour utilisation selon l'une quelconque des revendications précédentes dans laquelle l'utilisation a pour but d'inhiber ou de diminuer une dégradation de collagène dans un cartilage.

14. Composition pour utilisation selon l'une quelconque des revendications précédentes dans laquelle l'utilisation a pour but d'améliorer l'activité et/ou la mobilité d'un sujet.
